# EUROPEAN PATENT SPECIFICATION

(11) **EP 2 746 174 B1**
(45) Date of publication and mention of the grant of the patent: **29.06.2016**
(21) Application number: 12198586.5
(22) Date of filing: 20.12.2012
(51) Int. Cl.: B65B 55/08, A61L 2/08

(54) **Device and method for sterilizing packaging containers by electron beam**
Vorrichtung und Verfahren zur Sterilisierung von Verpackungsbehältern mit Elektronenstrahlen
Dispositif et procédé pour stériliser des récipients d'emballage par faisceau d'électrons

(43) Date of publication of application: 25.06.2014
(73) Proprietor: Tetra Laval Holdings & Finance S.A., 1009 Pully (CH)
(72) Inventor: Åkesson, Mats, 213 63 Malmö (SE); Dickner, Jonas, 253 51Påarp (SE)
(74) Representative: Tetra Pak - Patent Attorneys SE

(56) References cited:
- US-A1- 2012 219 455

## Description

### FIELD OF THE INVENTION

The present invention relates to a sterilization device for sterilizing packaging containers with electron beams in a filling machine. The invention also relates to a method.

### BACKGROUND OF THE INVENTION

Within the food industry, it is common practice to pack liquid and partly liquid food products in packaging containers manufactured from a packaging laminate comprising a core layer of paper or paperboard and one or more barrier layers of, for example, polymer material or aluminium foil.

An increasingly common packaging type is the "carton bottle" manufactured in a filling machine in that packaging blanks of the above-described packaging laminate are formed and sealed as a sleeve. Said sleeve is closed in one end in that a top of thermoplastic material is injection moulded directly on the sleeve end portion. The sheets of packaging laminate may be cut from a magazine reel of packaging laminate.

When the top is finished the packaging container is ready to be filled with product through the still open bottom, and then sealed and finally folded. Before the filling operation the packaging container undergoes treatment. If distribution and storage is to be made in chilled temperature the packaging container is disinfected, whereas if distribution and storage is to be made in ambient temperature, the packaging container needs to be sterilized. A conventional way of sterilizing a ready-to-fill packaging container is to use hydrogen peroxide, preferably in gas phase.

Another way to sterilize such packaging containers is to irradiate it by means of a low voltage electron beam emitted from an electron beam emitter. An example of linear irradiation by electron beam of ready-to-fill packaging containers is disclosed in the international patent publication WO 2005/002973. The electron beam emitter is cylindrical with an electron exit window positioned at one of the distal ends. The packaging container is lifted to surround the electron beam emitter during the sterilization cycle. Other examples of irradiation of packaging containers, in these cases PET bottles, are described in for example WO 2011/011079 and EP 2 371 397. In these systems emitters are used having a diameter small enough to be passed through a neck portion of the bottles.

Another example is the document US 2012/0219455 also describing irradiation of PET bottles. Here, the interior of the bottle is irradiated as well as a portion of the exterior; a neck portion above a neck ring. When entering a sterile chamber an electron cloud of the electron beam emitter is protecting the sterilized neck and the interior of the bottle.

### SUMMARY OF THE INVENTION

The present invention relates to a sterilization device for sterilizing packaging containers with electron beams in a filling machine. The sterilization device comprises at least one first electron beam emitter adapted for sterilization of at least the interior of the packaging container and at least one second electron beam emitter adapted for sterilization of at least the exterior of said packaging container. A sterilization chamber in which the first electron beam emitter is adapted to sterilize the interior of the packaging container. The at least one second electron beam emitter is positioned such that an electron exit window thereof is facing an interface region, said interface region forming an opening from the sterilization chamber to an aseptic chamber. An electron cloud emitted from the at least one second electron beam emitter during operation is adapted to form an aseptic lock at least covering the interface region.

In one embodiment an electron cloud emitted from the first electron beam emitter, upon finalisation of the interior sterilization of the packaging container, is adapted to be partly overlapping the electron cloud emitted from the at least one second electron beam emitter.

In one or more embodiments the sterilization chamber comprises at least first conveying means, and the aseptic chamber comprises at least second conveying means, and the first and second conveying means are separately arranged one on each side of the interface region.

In one or more embodiment the first conveying means is adapted to push the packaging container at least partly through the aseptic lock in the interface region, and release the packaging container when it has been received by the second conveying means on the other side of the aseptic lock.

In one or more embodiments the first electron beam emitter and the packaging container are adapted to perform a mutual relative movement during which the interior sterilization of the packaging container takes place.

In one or more embodiments the first conveying means is adapted to displace the packaging container in relation to the first electron beam emitter between a first position in which the packaging container and the first electron beam emitter are not engaged with each other and a second position in which the packaging container and the first electron beam emitter are fully engaged with each other.

In one or more embodiments said at least one second electron beam emitter being adapted to sterilize the exterior of the packaging container when passing the packaging container from the sterilization chamber to the aseptic chamber, through the interface region, such that the packaging container reaching the aseptic chamber is fully sterilized on its exterior.

In one or more embodiments it comprises two second electron beam emitters, arranged opposite each other with their electron exit windows facing each other and the interface region, in such a way that the packaging containers can pass in between them.

In one or more embodiments the first electron beam emitter is arranged in the first conveying means, and the first conveying means is adapted to convey the packaging container synchronously with the first electron beam emitter, keeping a longitudinal axis of the first electron beam emitter aligned with a longitudinal axis of the packaging container.

In one or more embodiments the first conveying means is adapted to displace the packaging container from a sterilization chamber in-feed position to an out-feed position, the out-feed position being in the vicinity of the interface region, and the first electron beam emitter is adapted to sterilize the interior of the packaging container at least during a portion of the displacement between the sterilization in-feed position to the out-feed position.

In one or more embodiments the first conveying means is provided with more than one first electron beam emitter.

In one or more embodiments the aseptic chamber comprises stations in which the packaging container is adapted to be filled with product and sealed.

The invention also comprises a method of sterilizing packaging containers with electron beams in a filling machine. The method comprises the steps of sterilizing at least the interior of the packaging container with a first electron beam emitter arranged in a sterilization chamber. It further comprises the step of sterilizing at least the exterior of the packaging container with at least one second electron beam emitter. The step of sterilizing the exterior of the packaging container is performed in an interface region, said interface region forming an opening from the sterilization chamber to an aseptic chamber, and wherein an electron cloud emitted from the at least one second electron beam emitter during operation is adapted to form an aseptic lock at least covering the interface region.

In one or more embodiments the method comprises the step of finalizing the interior sterilization such that an electron cloud emitted from the first electron beam emitter partly overlaps the electron cloud emitted from the at least one second electron beam emitter.

### BRIEF DESCRIPTION OF THE DRAWINGS

In the following, an embodiment of the invention will be described in greater detail, with reference to the enclosed schematic drawings, in which:
Fig. 1a is a view of a packaging container and an exemplary first electron beam emitter, for sterilizing the interior of the packaging container, in a fully engaged sterilization position,
Fig. 1b is a view of an alternative packaging container and first electron beam emitter,
Fig. 2 is a perspective view of a second electron beam emitter for sterilizing the exterior of the packaging container,
Fig. 3 is a perspective view of a cathode which may be used in the electron beam emitter of Fig. 2,
Fig. 4 is a cross section of the cathode of Fig. 3,
Fig. 5a is a cross sectional view illustrating a first embodiment of the invention, showing the general principle of the invention,
Fig. 5b is a schematic side view of a second electron beam emitter and its electron cloud,
Fig. 5c is a schematic front view of the second electron beam emitter of Fig. 5b and its electron cloud,
Fig. 5d is a view of a first electron beam emitter and its electron cloud,
Fig. 5e is a view of the volume of the interface region,
Fig. 6a is a perspective view illustrating a second embodiment of the invention,
Fig. 6b is a top view illustrating portions of the second embodiment of the invention,
Fig. 6c is a side view of two second electron beam emitters having electron exit windows being inclined relative each other,
Figs. 6d-6g are a cross sectional views of the aseptic chamber, a first electron beam emitter, a second electron beam emitter and a packaging container of the second embodiment,
Fig. 7a is a perspective view of a third embodiment of the invention,
Fig. 7b is top view illustrating portions of the third embodiment,
Fig. 7c is a side view of a first electron beam emitter, a second electron beam emitter and two packaging containers of the third embodiment, and
Fig. 7d is a perspective view of portions of the aseptic chamber of the third embodiment.

### DETAILED DESCRIPTION OF A PREFERRED EMBODIMENT

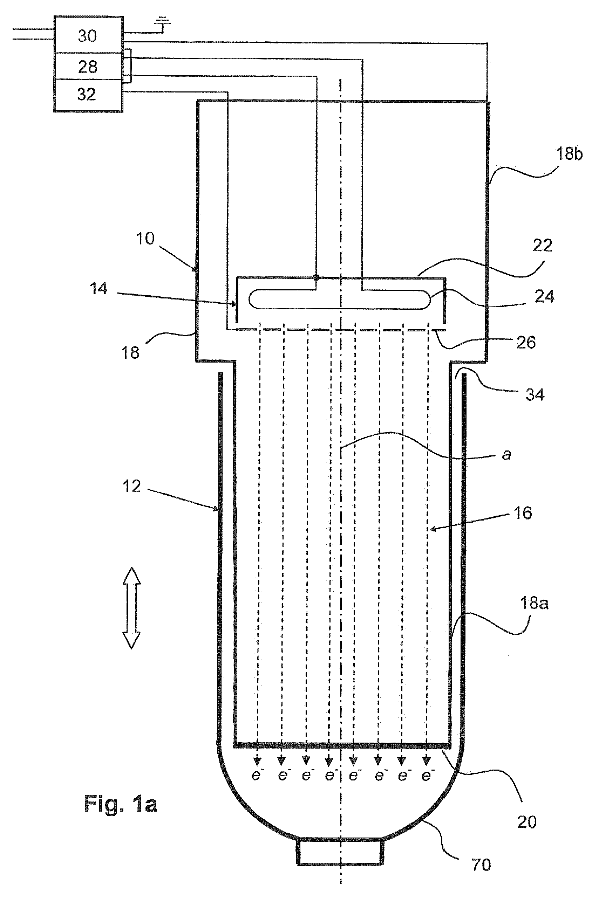

In the following, and with reference to Figure 1a, an exemplary first electron beam emitter 10 for sterilizing the interior of ready-to-fill packaging containers 12 and the concept of electron beam sterilization will be briefly described.

The electron beam emitter 10 comprises an electron generator 14 for emitting a substantially circular electron beam 16 along a path. The electron generator 14 is enclosed in a hermetically sealed vacuum chamber 18. Said vacuum chamber 18 is provided with an electron exit window 20.

The electron generator 14 comprises a cathode housing 22 and a filament 24. Optionally, the electron generator 14 also comprises a control grid 26. In use, an electron beam 16 is generated by heating the filament 24. When an electrical current is fed through the filament 24, the electrical resistance of the filament 24 causes the filament to be heated to a temperature in the order of 2000°C. This heating causes the filament 24 to emit a cloud of electrons. The electrons are accelerated towards the electron exit window 20 by means of a high-voltage potential between the cathode housing 22 and the exit window 20 (being the anode). Further, the electrons pass through the electron exit window 20 and continue towards the target area, i.e. in this case the inside of the packaging container 12.

The high-voltage potential is created by for example connecting the cathode housing 22 and the filament 24 to a power supply 28 and by connecting the vacuum chamber to ground 30. The first electron beam emitter 10 is generally denoted low voltage electron beam emitter if the voltage is below 300 kV. In the disclosed design the accelerating voltage is in the order of 95 kV. This voltage results in a kinetic (motive) energy of 95 keV in respect of each electron. However, another voltage can be chosen, for example in the interval 75-150 kV. By applying an electrical potential also to the control grid 26 the emission of electrons may be further controlled. If a separate and variable electrical potential is applied to the control grid 26 it makes it possible to use the control grid 26 for active shaping of the generated electron beam. For these purposes the control grid 26 may be electrically connected to a separate power supply 32.

The filament 24 can be made of tungsten. The grid 26, placed between the filament 24 and an electron beam exit window 20 provided with a number of openings and is used for diffusing the electron beam 16 into a more uniform beam, and for focusing the electron beam 16 towards the target area.

The emitter 10 is, as mentioned, further provided with an electron exit window 20. The window 20 can be made of a metallic foil, such as for example titanium, and can have a thickness in the order of 4-12 µm. A supporting net (not shown) formed of aluminum or copper supports the foil from inside the vacuum chamber 18. The electrons are exiting the vacuum chamber 18 through the exit window 20.

In this embodiment the vacuum chamber 18 is made up of two cylindrical bodies 18a, 18b with substantially circular cross sections. An end of the first cylindrical body 18a is provided with the electron exit window 20. The diameter of said first body 18a is small enough to be inserted into the ready-to-fill packaging container 12, the cross section of said first body is dimensioned such that it can be guided through an opening 34 of the packaging container 12. The second body 18b is provided with the electron beam generator 14, and the diameter of said second body 18b is larger than the first body 18a. The diameter of the emitted electron beam 16, while still inside the emitter 10, is smaller than the diameter of the first body 18a.

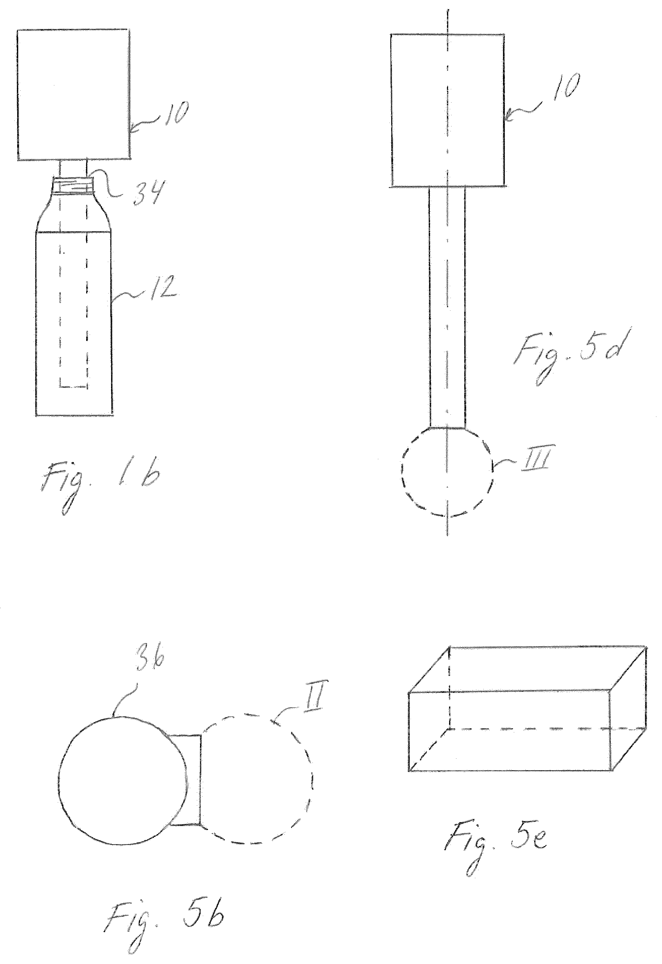

In Figure 1a the opening 34 of the packaging container is an open bottom end, which after filling will be sealed and folded to form a substantially flat bottom surface. It should however be understood that the opening, through which the first electron beam device is received, may in other embodiments be arranged in the top of the packaging container, constituting a neck or spout portion of the packaging container. Figure 1b illustrates such. The neck or spout portion will, after filling, be sealed by for instance a screw cap.

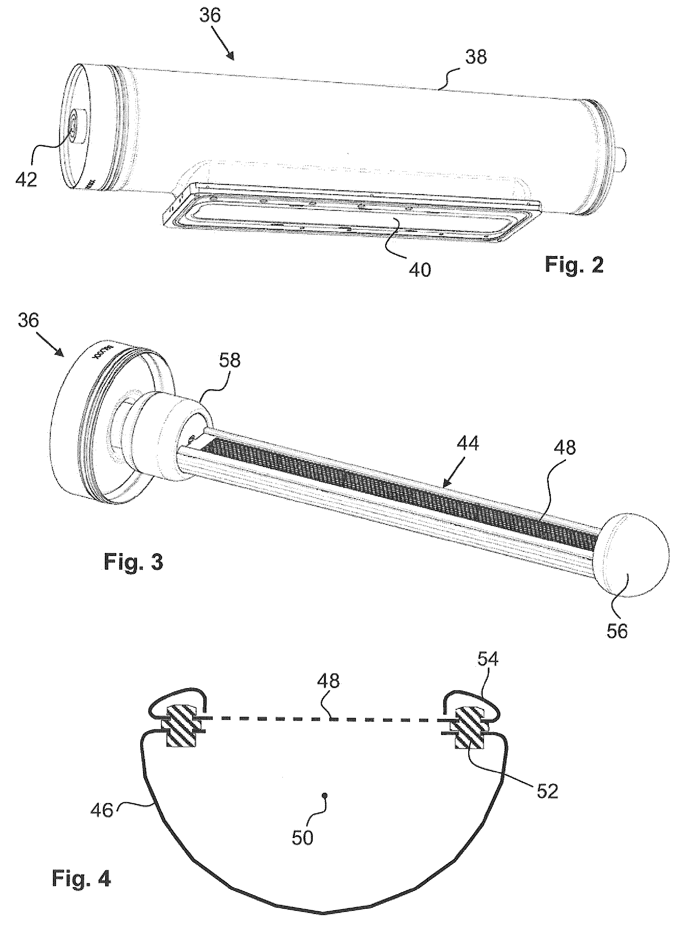

Fig. 2 is a perspective view of an exemplary hermetically sealed second electron beam emitter 36 for sterilizing the exterior of ready-to-fill packaging containers 12. The purpose of the drawing is simply to illustrate the basic components of the emitter, and it should be emphasized that the purpose is not to provide a true constructional drawing or in any other way limit the present invention.

The main component of the electron beam emitter is the tubular body 38, which has an elongate shape. An electron exit window 40 provides an outlet for electrons from the vacuum inside the tubular body 38. The exit window 40 in turn comprises subassemblies not relevant for the present invention, yet having the properties of providing an outlet window for electrons while preserving vacuum inside the body 38. A proximal end of the body 38 comprises an assembly including electrical connections 42.

Fig. 3 shows the emitter 36 without the tubular body 38, and a cathode 44 is shown. The cathode 44 comprises a cathode housing 46, which is also shown in the very schematic cross section of Fig. 4. The cathode housing 46 is formed as a semi-annular shell, the open side of which is covered by a control grid 48. Inside the annular shell of the cathode housing 46 one or more filaments 50 (see Fig. 4) are arranged, extending from a proximal end of the cathode housing 46 to a distal end thereof. In use, an electron beam is generated by heating the filament 50, using a current, and by accelerating the electron towards the electron exit window 40 by means of a high-voltage potential between the cathode housing 46 and the exit window 40 (being the anode). The high-voltage potential is created by for example connecting the cathode housing to a power supply and by connecting the tubular body to ground. By applying an electrical potential also to the control grid 48 the emission of electrons may be further controlled. This can be achieved by connecting the control grid 48 to a separate power supply.

The second electron beam emitter 36 has an accelerating voltage in the order of 95 kV. This voltage results in a kinetic (motive) energy of 95 keV in respect of each electron. However, another voltage can be chosen, for example in the interval 75-150 kV.

The control grid 48 comprises a flat perforated surface comprising a pattern of openings or through-holes for passage of electrons. The open side of the cathode housing 46, carrying the control grid 48, should for obvious reasons be facing the electron exit window 40. The cathode housing 46 and the control grid 48 are mounted together by means of attachment means 52. If there is a difference in electrical potential between the cathode housing 46 and the grid 48 said attachment means 52 are preferably electrical isolator elements. Free longitudinal end portions 54 of the control grid 48 are bent in a direction towards each other, i.e. in a lateral direction being perpendicular to the extension of the longitudinal end portions, to form bulge-like shapes for the formation of electron beam shaping electrodes. Such electrodes are sometimes referred to as "Wehnelt" electrodes. The bulge-like shape will assist in the generation of a smooth predictable electrical field to the benefit of performance of the electron beam emitter. They help shaping the electric field so that the electrons will hit the exit window 40 in an essentially right angle, i.e. in a direction essentially perpendicular to the plane of the exit window 40.

The described cathode is fitted into the electron beam emitter as shown in Fig. 3. The proximal end as well as the distal end of the cathode housing 46 comprises electrical connections as well as physical suspensions for the filament 50. At the distal end this arrangement is housed inside or covered with a dome-shaped cap 54. At its proximal end the cathode housing 46 is suspended to the elongate body and the suspension is encapsulated by an annular cover 58.

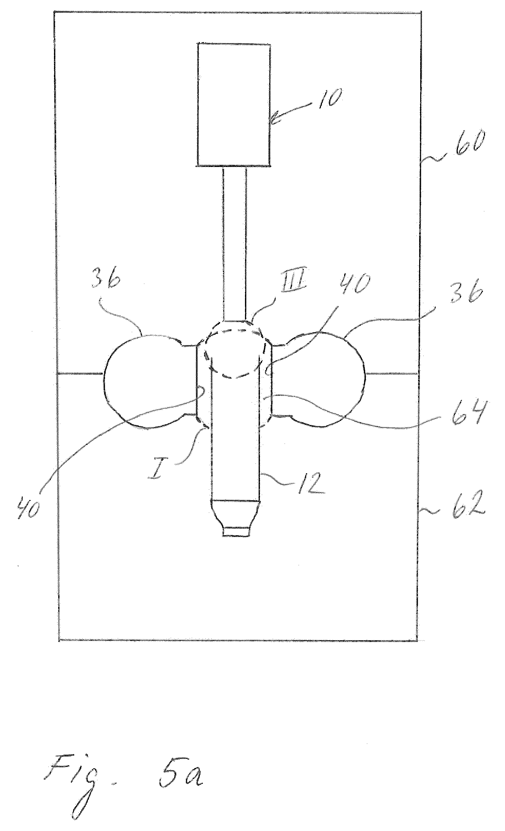

Figures 5a-5e show a first embodiment illustrating the general concept of the invention. In Fig. 5a a sterilization chamber of a filling machine is shown. The sterilization chamber is denoted with reference numeral 60. In the sterilization chamber 60 at least one first electron beam emitter 10 is arranged on a first conveying means (not shown). The first electron beam emitter 10 is for example of the type described above in relation to Figure 1a, and it is adapted for sterilization of at least the interior of the packaging container 12. The interior of the packaging container 12 is all inside surfaces of the packaging container. The sterilization chamber 60 is not kept sterile due to the fact that non-sterilized packaging containers 12 are continuously fed into the chamber for the purpose of becoming sterilized. However, near the sterilization chamber 60 there is provided an aseptic chamber 62. The aseptic chamber 62 is arranged downstream the sterilization chamber 60, i.e. the packaging containers 12 will first enter the sterilization chamber 60 and then subsequently enter the aseptic chamber 62. During production of packaging containers 12 the environment in the aseptic chamber 62 should be sterile, i.e. free from dirt and microbiological material. The aseptic chamber 62 comprises stations (not shown) in which the packaging container 12 is adapted to be filled with a product, such as for example a beverage, and sealed. Depending on which end of the packaging container that is open, the bottom as shown in Fig. 1a, or the neck as shown in Fig. 1b the sealing station may look different. In the case of filling into an open bottom end the sealing station comprises sealing bars for heatsealing the packaging material. In the case of filling through a spout in a neck region of a packaging container the sealing station instead comprises a capping station. Before the packaging containers 12 have been sealed it needs to be ensured that the environment around them is sterile, that the packaging containers entering the aseptic chamber 62 from the sterilization chamber 60 are sterile, and that no contaminated air is allowed to escape into the aseptic chamber 62 from the sterilization chamber 60.

Between the sterilization chamber 60 and the aseptic chamber 62 there is an interface region 64 through which the packaging containers 12 can pass from the sterilization chamber 60 to the aseptic chamber 62, i.e. the interface region 64 forms an opening 64 between the sterilization chamber 60 and the aseptic chamber 62. Hence, the interface region 64 is an open passage between the sterilization chamber 60 and the aseptic chamber 62. In the vicinity of the interface region 64 there is provided at least one second electron beam emitter 36 adapted for sterilization of at least the exterior of the packaging container 12. The exterior of the packaging container is all surfaces on the outside of the packaging container.

In this specific embodiment there are two second electron beam emitters 36 arranged in the interface region 64. They are arranged opposite each other, with the interface region 64 in between them, such that the packaging containers can pass through it. Further, their electron exit windows 40 are facing the interface region 64 such that the second electron beam emitters 36 are adapted to sterilize the exterior of the packaging containers when those are passed from the sterilization chamber 60 to the aseptic chamber 62 through the interface region 64.

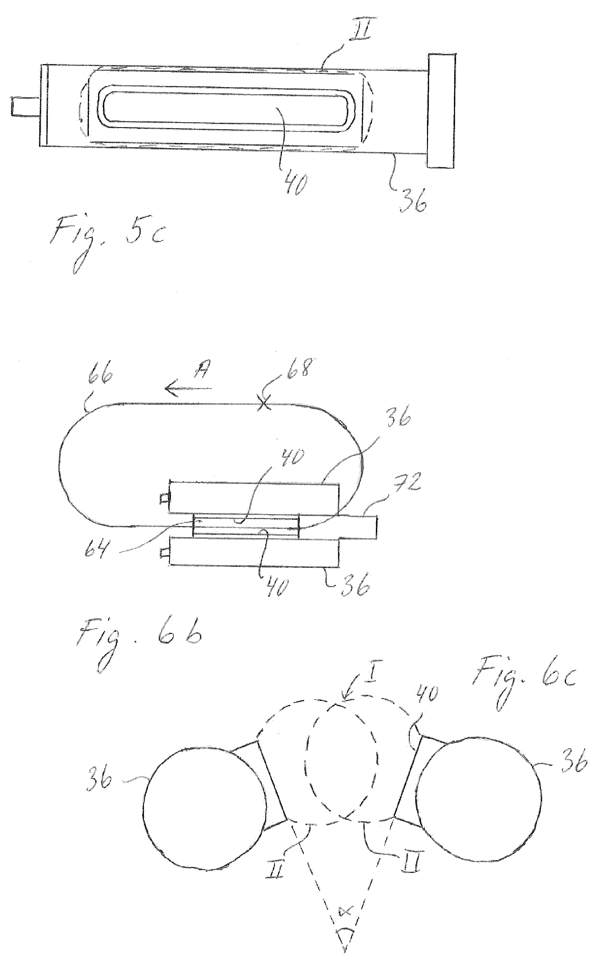

The electron beam generated by each of the second electron beam emitters 36 during operation form a combined electron cloud. The boundary of the cross section of that electron cloud is shown with dashed line I in Figure 5a. The electron cloud of one of the second electron beam emitters 36 is shown in Figure 5b and 5c, and is denoted II. It has a substantially circular cross section. The combined electron cloud I fills the interface region 64 forming an aseptic lock. The electron cloud I has an extension in three dimensions and forms a volume being large enough to at least cover the interface region 64. Figure 5e illustrates the volume of the interface region 64. The longer sides are approximately as large as the longitudinal extension of the electron exit window 40 of the second electron beam emitter. The volume of the electron cloud I is at least as large as the volume of the interface region 64, i.e. the cloud I at least fills the volume of Fig. 5e. Within the boundaries of the electron cloud I, i.e. the aseptic lock, the electron beams have at least enough energy to kill any microbiological material travelling through the interface region 64 at the same velocity as the velocity of the packaging containers or of any particle or air flow travelling through the same. Typically, for a second electron beam emitter 36 of the above described type, the dose rate at the boundary of the electron cloud II is approx. 400-800 kGy/s. In the centre of the electron cloud the dose rate is higher. The size of the interface region 64 and the energy of the second electron beam emitter will need to be matched for each application such that the electron cloud at least covers the interface region and such that the minimum dose rate of the electron cloud I is enough to kill any microbiological particles passing the interface region 64 at a chosen maximum velocity.

The electron cloud I, i.e. the aseptic lock, is suitable for killing microbiological material within it's direct reach. The innermost interior of the packaging container 12 is not within direct reach, it is "shadowed" by the packaging material of the packaging container. Hence, to ensure that the sterile environment inside the aseptic chamber 62 is not contaminated through the interface region 64 it is important that also the interior of the packaging containers is sterile when entering the aseptic lock, or that any still unsterile portion of the interior of the packaging container is protected by an electron cloud from the first electron beam emitter 10.

Figure 5a shows the case in which the interior of the packaging container 12 is sterile upon entering the aseptic chamber 62. This gives a simple mechanical solution since the first electron beam emitters 10 don't need to enter the aseptic chamber 62. By letting the electron cloud III of the first electron beam emitter 10, upon finalising the interior sterilization of the packaging container 12, at least partly overlap the electron cloud I of the second electron beam emitters, it can then be ensured that every portion of the packaging container 12 reaching the inside of the aseptic chamber 62 has been fully sterilized. In other words, by creating electron clouds I, III being partly overlapping it is ensured that any microbiological material on the inside surface of the packaging container cannot escape to the outside surface, or vice versa, without being killed. Yet, the first electron beam emitters 10 and any conveying means need not enter the aseptic chamber 62.

A cross section of the electron cloud III of the first electron beam emitter 10 is represented by a dashed line III, see Figure 5d. The cross sectional shape is circular or droplet-shaped. The electron cloud is axis-symmetrical and the cloud volume is thereby spherical or droplet-shaped. Within the dashed line III the electron beam has at least enough energy to kill any microbiological material travelling through the interface region at the same velocity as the velocity of the packaging containers or of any air flow travelling through the same. Typically, for a first electron beam emitter 10 of the above described type, the dose rate at the boundary of the electron cloud III is approx. 1000-1600 kGy/s. In the centre of the electron cloud the dose rate is higher. The energy of the first electron beam emitter 10 needs to be matched with the sterilization time available and the packaging container size and shape.

It should be pointed out that also the electron cloud III forms an aseptic lock, but an aseptic lock inside the packaging container 12 during sterilization. Although most of the interior sterilization is made in the sterilization chamber 60, not being fully sterile as compared with the aseptic chamber 62, the electron cloud III is able to form an aseptic lock inside the packaging container. No dirt or microbiological material or particle can manage to travel through the cloud III and into the sterilized interior of the packaging container 12. The volume of the electron cloud III of the first emitter 10 covers the opening 34 of the packaging container. Hence, the sterility of the interior, below the electron cloud III, can be assured even during the time when the packaging container 12 is still in the sterilization chamber 60.

Figure 5a shows the moment when the packaging container 12 is on its way through the interface region 64 between the two opposite second electron beam emitters 36. The packaging container 12 is on its way to the first position, meaning that the packaging container and the first electron beam emitter 10 will soon no longer be engaged with each other. Still, the uppermost end of the packaging container, being the open bottom end 34 of the packaging container 12, is still affected by the electron cloud III from the first electron beam emitter 10, although the first electron beam emitter 10 is no longer inside the packaging container 12. At the same time the packaging container 12 has been passed into the electron cloud I of the second beam emitters 36, and most of the exterior of the packaging container 12 has already been sterilized by the electron cloud I of the second beam emitters 36. The packaging container 12 is moved through the interface region 64 with its top and screw cap first. In Figure 5a it can be seen that the electron cloud III of the first electron beam emitter 10 partly overlaps the electron cloud I of the second electron beam emitters 36. The overlapping is created in the area of the uppermost end of the packaging container, i.e. at the open bottom end 34 of the packaging container 12. When the packaging container 12 reaches the aseptic chamber 62 it is fully sterilized.

With Figures 5a-5e the general principle of the aseptic lock has been described. By creating a sterile airflow through the interface region 64, in a direction from the aseptic chamber 62 towards the sterilization chamber 60, the aseptic lock can be sustained even if the second beam emitters 36 are temporarily shut off. Of course the packaging container transport needs to be stopped as well, since no sterilization will take place.

What is not shown in Figure 5a is that the sterilization chamber 60 is provided with first conveying means adapted to push the packaging container 12 at least partly through the aseptic lock in the interface region 64. In the aseptic chamber 62, on the other side of the aseptic lock, there are second conveying means (not shown) that will receive the packaging container 12. When received in the second conveying means the packaging container will be released by the first conveying means. Alternatively, the first conveying means is releasing the packaging container before the second conveying means grip it, having the packaging container falling freely for a moment.

The first conveying means in the sterilization chamber 60 is arranged separately from the second conveying means. The first and second conveying means are separately arranged one on each side of the interface region 64. Hence, there is only necessary to machine-sterilize the second conveying means in the aseptic chamber 62. The first conveying means are gripping the packaging container on the exterior surface. To obtain a fully sterilized packaging container the first conveying means must release the packaging container within the electron cloud I of the second electron beam emitters 36, or within reach of the electron cloud III of the first electron beam emitter 10, to have also the contact surface sterilized. The contact surface is the surface at which the gripping means has gripped the packaging container. Alternatively, the first conveying means are gripping the packaging container on its interior surface. To obtain a fully sterilized packaging container the first conveying means must release the packaging container within the electron cloud III of the first electron beam emitter 10.

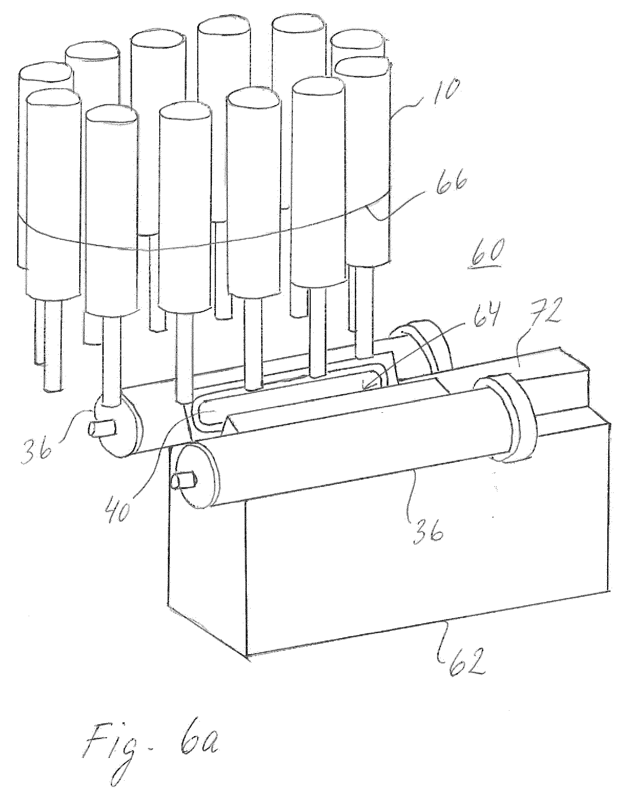

Figures 6a-6h show a more detailed, second embodiment of the invention. In the following description of the seond embodiment some of the reference numerals used for describing the first embodiment will be re-used for similar features.

In this second embodiment several first electron beam emitters 10, of the type described above with reference to Fig. 1, are arranged on first conveying means, see Fig. 6a. The first conveying means comprises a drive belt or chain on which the first electron beam emitters 10 are attached. The belt or chain is just illustrated by line 66. This belt or chain is adapted to convey each packaging container synchronously with a first electron beam emitter 10, keeping a longitudinal axis of the first electron beam emitter aligned with a longitudinal axis of the packaging container, see common longitudinal axis *a* in Fig. 1a. In this embodiment the drive belt or chain 66 of the first conveying means is shaped with two opposite linear portions and two opposite curved portions, see Fig. 6b. The movement of the belt or chain is continuous, but alternatively it is intermittent. The direction of the movement is illustrated by the arrow A.

The first conveying means is adapted to displace the packaging container from a sterilization chamber in-feed position 68 to an out-feed position, the out-feed position being near the interface region 64, at a position slightly above. The in-feed position 68 is located at one of the linear portions of the drive belt or chain 66, whereas the out-feed position is located at the other linear portion, see Fig. 6b. The first electron beam emitter 10 is adapted to sterilize the interior of the packaging container at least during a portion of the displacement from the sterilization in-feed position 68 to the out-feed position.

The first conveying means comprises gripping means, not shown, adapted to grip the packaging container at the in-feed position 68. The packaging container is there started being displaced towards the first electron beam emitter. Since the first electron beam emitter 10 is aligned with the opening 34 of the packaging container 12 the electron beam emitter 10 is received in the packaging container 12. Hence, sterilization of the interior of the packaging container is commenced. Somewhere between the in-feed position 68 and the out-feed position the packaging container has been displaced such that the packaging container is fully engaged with the first electron beam emitter. The fully engaged second position is shown in Fig. 1a. In that position the innermost area of the packaging container 12 may be sterilized, in this case a top portion 70 of the packaging container 12.

The interior sterilization cycle is completed when the packaging container 12 reaches the out-feed position near the interface region 64. When the packaging container 12 reaches said outfeed position, the packaging container is retracted, or has already been retracted from the second position back to the first position. The packaging container is then ready to be fed out from the sterilization chamber and into the aseptic chamber 62.

For achieving the relative vertical movement towards and away from the electron beam emitters the packaging conveying means may comprise a cam curve, servo motor or similar that guides the gripping means in the vertical direction upon rotation of the belt or chain. Further, the gripping means are preferably gripping the packaging container near the opening 34, on the outside surface, by for example a clamping force. However, since the gripping means as such are not the focus of this invention, they will not be described further.

The out-feed position is, as mentioned before, arranged near the interface region 64, slightly above it. On each side of the opening, parallel to the longitudinal direction of the opening, two second electron beam emitters 36 are arranged. They are for example of the type described in relation to Figs. 2-4. They are arranged opposite each other with their electron beam exit windows 40 facing each other and the interface region 64. Either the planes of the electron exit windows 40 are parallel to each other, or slightly inclined in relation to each other as shown in Fig. 6c. In the latter case there is an angle α between the two electron exit windows 40.

Figure 6b shows a top view of the interface region 64, and the two second electron beam emitters 36. Here the electron exit windows 40 are shown as being parallel to each other. The interface region 64, being the only entrance to the aseptic chamber 62 from the sterilization chamber 60, will during operation be totally covered by the electron cloud I of the second emitters. A wall 72, to the right of the electron cloud I, protects the upper portion of the aseptic chamber 62.

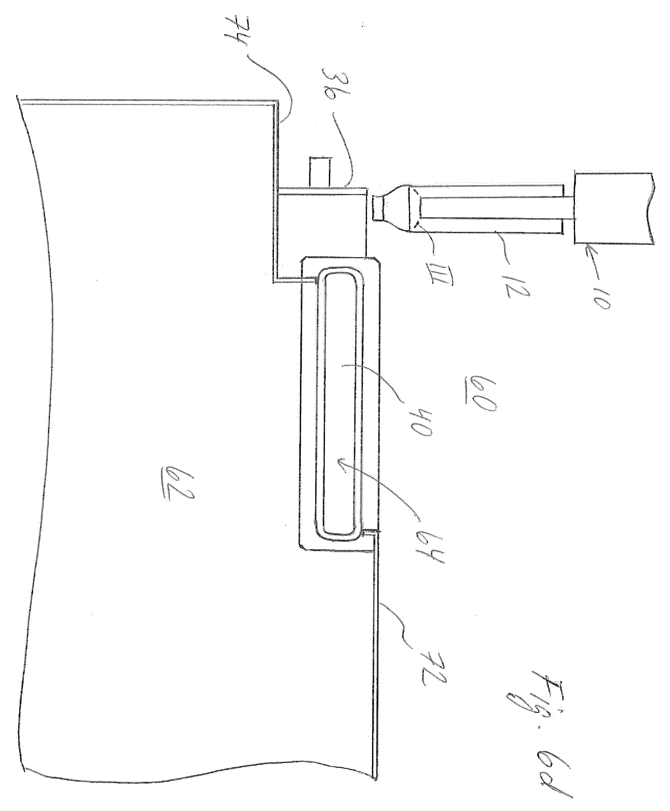

Figures 6d-6g show a cross section through the interface region 64, and a portion of the aseptic chamber 62. Hence, only one second electron beam emitter 36 is shown. The figures further show one first electron beam emitter 10 and one packaging container 12. By means of the Figures 6d-6g the movement of the first electron beam emitter 10 and the packaging container 12 through the interface region 64 will be described.

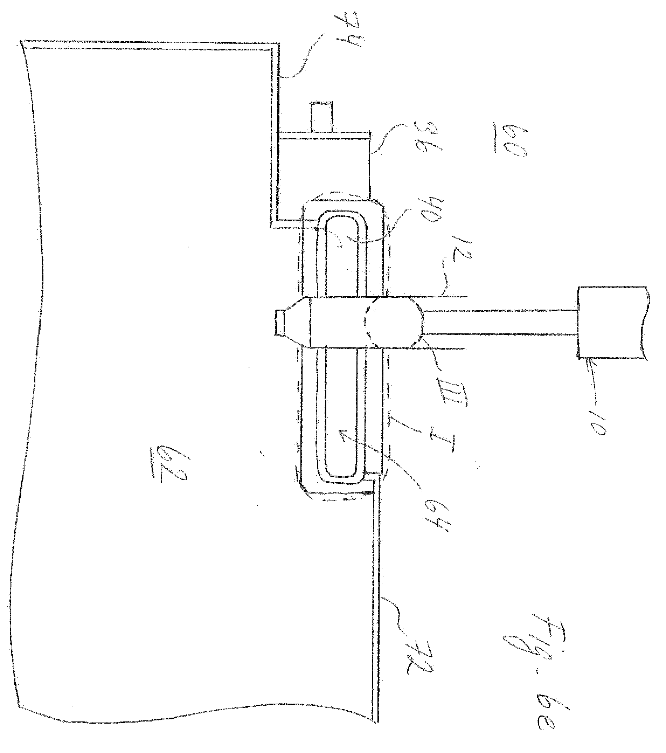

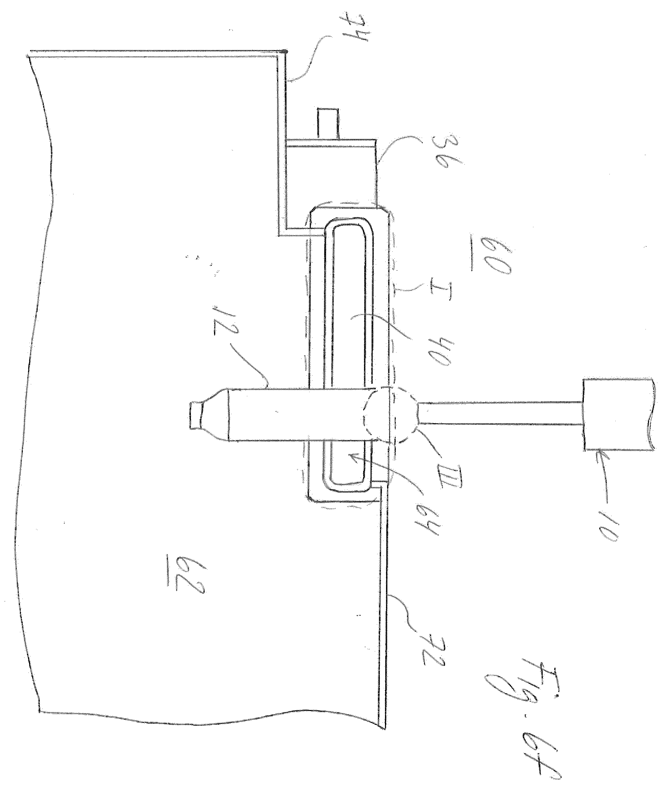

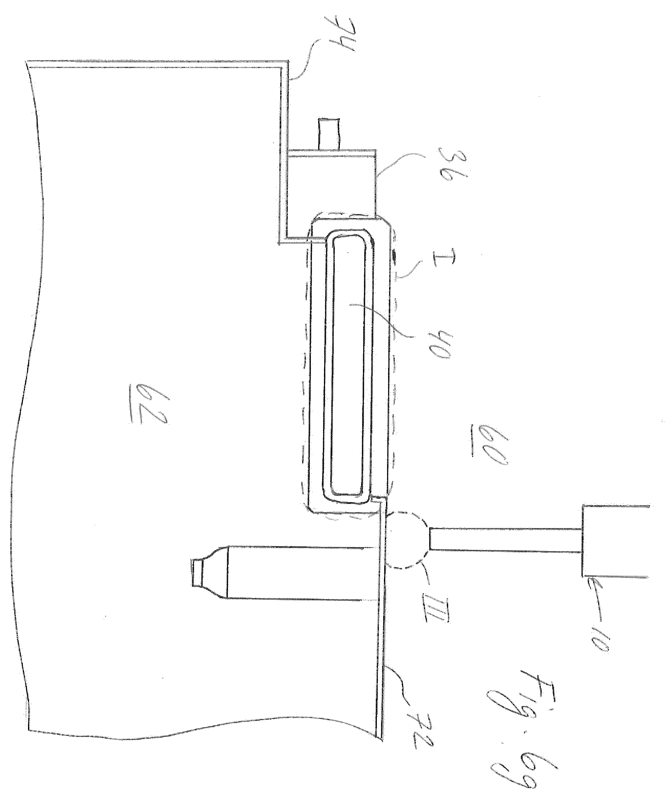

Figure 6d shows the second postion in which the packaging container 12 is fully engaged with the first electron beam emitter 10. The first electron beam emitter 10 and the packaging container 12 have moved from the in-feed position 68 to a position slightly to the left of the interface region 64. Further, as can be seen in the figure, the first electron beam emitter 10 and the packaging container 12 is positioned slightly above the second electron beam emitters 36. Two walls of the aseptic chamber 62 are shown and denoted 72 and 74. In Figure 6e the first electron beam emitter 10 together with the packaging container 12 has moved a distance to the right by means of the belt or chain of the first conveying means. Further, the conveying means has started to vertically displace the packaging container 12 downwards in relation to the first electron beam emitter 10, from the second position to a position closer to the first position. The interior sterilization is on-going. The downward movement has further displaced the packaging container 12 into the electron cloud I of the second electron beam emitters 36, and the packaging container top 70, comprising the screw cap, is already through the interface region 64 and in the aseptic chamber 62. That portion has been sterilized on the outside by the second electron beam emitters 36. In Figure 6f the first electron beam emitter 10 and the packaging container 12 have been moved further to the right. The first electron beam emitter 10 is finalizing the interior sterilization, i.e. only the electron cloud III of the first electron beam emitter 10 is engaged with the opening 34 in the bottom end of the packaging container 12. In this moment the electron cloud III of the first electron beam emitter 10 is partly overlapping the electron cloud I of the second electron beam emitters. The gripping means of the first conveying means, positioned in the sterilization chamber 60, has released the packaging container 12, i.e. it is no longer gripping the packaging container. The gripping means is not passing the interface region 64 in this embodiment. Instead, the second conveying means, not shown, of the aseptic chamber 62 is within reach of the packaging container 12 and has taken over the gripping of the packaging container. Finally, Figure 6g shows a moment in which the packaging container 12 is fully sterilized and has been conveyed to the right in the figure by means of the second conveying means. The upper wall portion 72 of the aseptic chamber 62 is protecting the packaging container 12. On the other side of said wall 72 the first electron beam emitter 10 is conveyed, by the first conveying means, back to the in-feed position. On its way back to the in-feed position 68 the first electron beam emitter 10 moves past a sensor (not shown) for measurement of at least one dose control parameter of the electron beam, such as for example the dose rate (kGy/s).

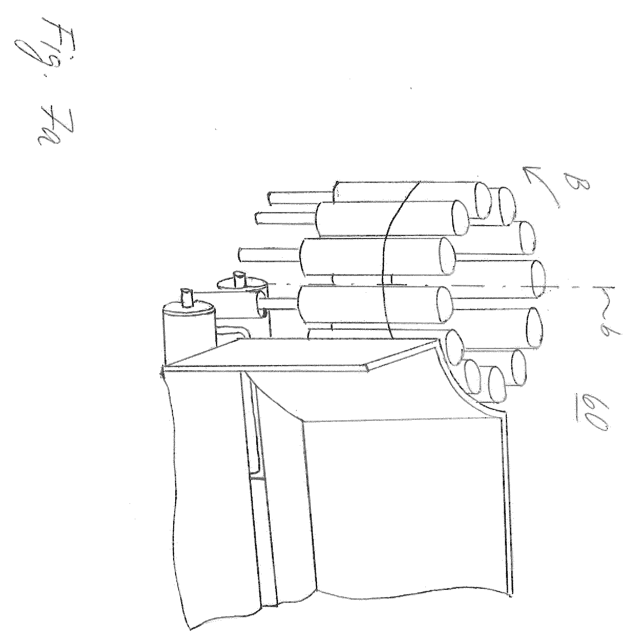

Figures 7a-7d show a more detailed, third embodiment of the invention. In this third embodiment some of the reference numerals used for describing the second embodiment will be re-used for similar features. Only the differences between the third and second embodiment will be described.

Figure 7a shows a sterilization device provided with several first electron beam emitters 10 of the type used for interior sterilization of packaging containers 12. Such first electron beam emitters 10 were described above with reference to Fig. 1a.

The first electron beam emitters 10 are provided to first conveying means. The first conveying means comprises a rotatable carrier, shown as a line 76. The rotatable carrier is, in this embodiment, a wheel and is rotatable round a centre shaft, here illustrated as a dash-dotted line b. The direction of the rotation is illustrated by the arrow B and the rotatable movement is continuous. The first emitters 10 are equally distributed to the periphery of the carrier 76, and are fixed to the carrier so that they are being carried along when the carrier 76 rotates. The transportation of the packaging containers 12 is made in a direction transverse to the longitudinal extension of the emitters 10.

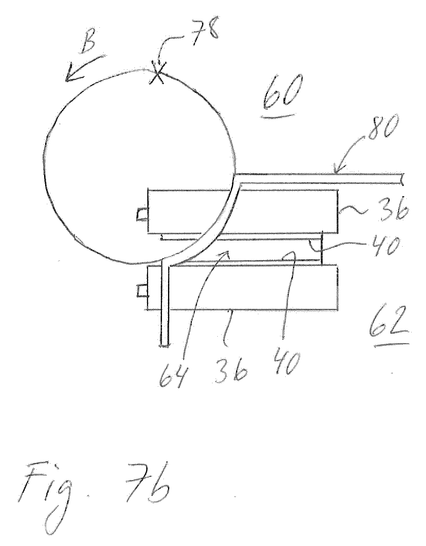

The circle of Figure 7b shows, very schematically, the transportation path of the packaging container and the first electron beam emitter. The first conveying means, not shown, is adapted to convey the packaging container from an in-feed position 78 in the sterilization chamber 60 to an outfeed position, said out-feed position being close to the interface region 64 towards the aseptic chamber 62. The conveying of the packaging container is made synchronously with the carrier revolution movement and in alignment with the electron beam emitter 10. A longitudinal centre axis of the packaging container is aligned with a longitudinal centre axis of the electron beam emitter, see common axis *a* in Fig. 1a. Further, the opening 34 in the packaging container 12 is concentric with the longitudinal centre axis of the packaging container.

The first conveying means is being further adapted to vertically displace the packaging container 12 in relation to the first electron beam emitter 10. In the embodiment shown the electron beam emitters 10 are arranged stationary in the carrier 76 and cannot move towards the packaging container. Due to the heavy weight, the fragile electron exit window and the high voltage connections it is an advantage to have the first electron beam emitters 10 stationary, and move the packaging containers 12.

The first conveying means displaces the packaging container 12 between a first position in which the packaging container 12 and the electron beam emitter 10 are not engaged with each other and a second position in which the packaging container 12 and the electron beam emitter 10 are fully engaged with each other. When the packaging container 12 and the electron beam emitter 10 are engaged the packaging container 12 has been raised to a position in which it surrounds the electron beam emitter 10 and the electron beam emitter 10 is sterilizing the interior of the packaging container. When they are not engaged the packaging container is positioned underneath the electron beam emitter, i.e. the packaging container has not started to surround the emitter 10, or has just been displaced down from the engaged position. At the in-feed position 78 and out-feed position the packaging container 12 is positioned in the first position, i.e. not in engagement with the electron beam emitter 10.

At the in-feed position 78 the packaging containers 12 are supplied to the sterilization device in the sterilization chamber 60. Each packaging container 12 is aligned with a corresponding electron beam emitter 10 and is gripped by means of gripping means (not shown). The gripping means is comprised in the first conveying means. The gripping means are attached to a drive belt or chain (not shown). The gripping means preferably grips the packaging container 12 in the top portion 70.

When the carrier 76 of the first conveying means rotates, so that the electron beam emitter 10 and packaging container 12 rotates from the in-feed position 78 to the interface region 64, the gripping means displaces the packaging container 12 towards the first electron beam emitter 10 for performing interior sterilisation.

Figures 7a and 7b further show two second electron beam emitters 36 for example of the type described in relation to Figs. 2-4. Similar to the arrangement of the two second electron beam emitters of the second embodiment, these two electron beam emitters 36 are arranged one on each side of the interface region, parallel to the longitudinal direction of the opening. They are arranged opposite each other with their electron beam exit windows 40 facing each other and the interface region. The planes of the electron exit windows are parallel to each other, but may alternatively be inclined as described in relation to Fig. 6c.

Figure 7a further shows a part of the aseptic chamber 62. The only entrance to the aseptic chamber 62 from the sterilization chamber 60 is the interface region 64. Some of the walls, together denoted 80, needed towards the sterilization chamber 60 are shown in figure. It is however to be understood that not all necessary walls are shown, and that the aseptic chamber 62 is closed except for the opening 64 towards the sterilization chamber 60 and a packaging container outlet (not shown) for out-feed of finished packaging containers 12.

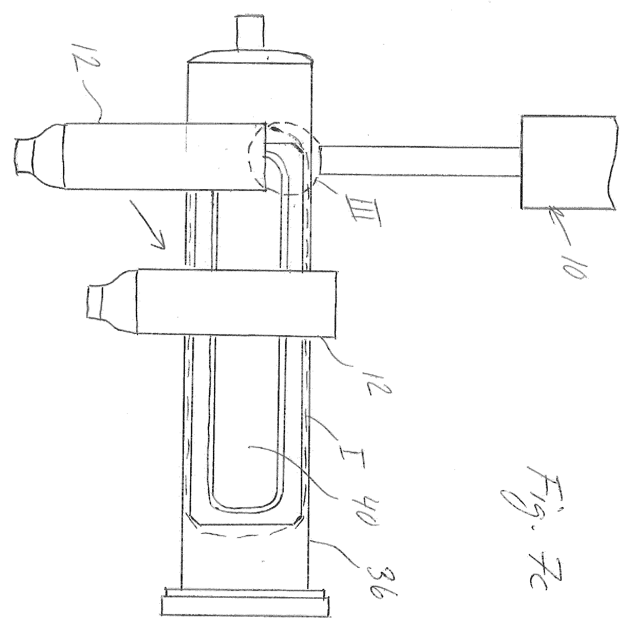

Figures 7c show a cross section through the opening in the interface region 64. Hence, only one second electron beam emitter 36 is shown. The figure further show two subsequent packaging containers 12 and one first electron beam emitter 10.

By means of the Figures 7a-7d the movement of one first electron beam emitter 10 and the packaging container 12 through the interface region 64 will be described.

When the packaging containers 12 has reached the position shown in Figure 7a it has been conveyed from the in-feed position 78 and almost to the out-feed position, i.e. the interface region 64. The packaging container 12 is being displaced downwards by the gripping means, from the second position to the first position, and the interior sterilization is on-going.

In Figure 7c the left packaging container 12 has been displaced further downwards and the interior sterilization is about to be finalised. Only the electron cloud III of the first electron beam emitter 10 is engaged with the opening 34 in the bottom end of the packaging container 12. At the same time the packaging container 12 as well as the first electron beam emitter 10 has been conveyed closer to the out-feed position, i.e. to the right in the figure and i.e. closer to the opening 64 towards the aseptic chamber 62. When the left packaging container 12 is moved to the right into the electron cloud I of the second electron beam emitters 36 the electron cloud III of the first electron beam emitter 10 will be partly overlapping the electron cloud I of the second electron beam emitters 36. Upon further displacement to the right, see the right packaging container 12, the gripping means will raise the packaging container so that it will move through the opening and the electron cloud I of the second electron beam emitters 36, i.e. through the aseptic lock, and become sterile on its outside surface. Before the top portion 70 of the packaging container 12 is moved into the aseptic lock/interface region 64 the gripping means of the first conveying means (not shown) releases the packaging container, and second conveying means (not shown) in the aseptic chamber 62 grips the packaging container and takes over the conveyance. In Figure 7c no walls of the aseptic chamber is shown.

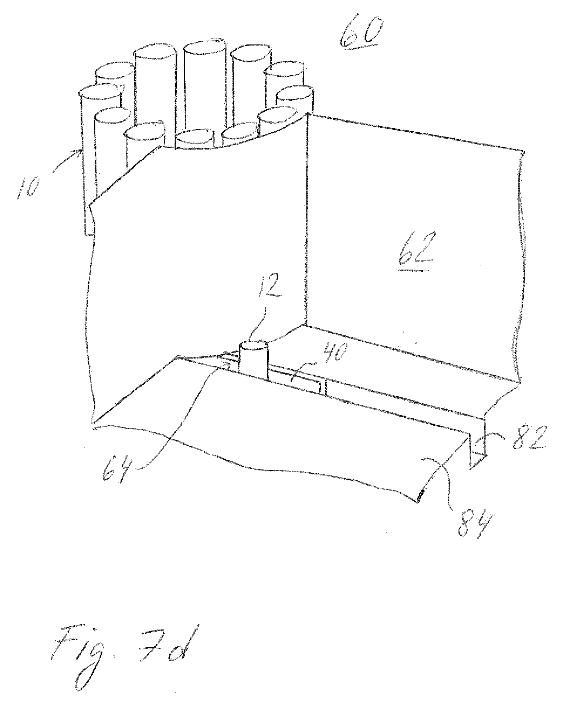

Figure 7d shows the interface region 64, i.e. the passage into the aseptic chamber 62 and a portion of a packaging container 12 being raised through the interface region. The figure also shows the walls 80 around the opening 64 as well as a ditch 82 in the lowermost wall portion 84 for receiving the top portion 70 of the packaging container 12 upon further movement up through the opening and into the aseptic chamber 62.

The invention comprises a method of sterilizing packaging containers with electron beams in a filling machine. The method has to a large extent already been described in relation to the various embodiments, and will only be summarized in the following. The method comprises the steps of sterilizing at least the interior of the packaging container with a first electron beam emitter arranged in a sterilization chamber, and sterilizing at least the exterior of the packaging container with a second electron beam emitter. The step of sterilizing the exterior of the packaging container is performed in an interface region, said interface region forming an opening from the sterilization chamber to an aseptic chamber. An electron cloud emitted from the second electron beam emitter during operation is adapted to form an aseptic lock at least covering the opening in the interface region. The method further comprises the step of finalizing the interior sterilization such that an electron cloud emitted from the first electron beam emitter partly overlaps the electron cloud emitted from the second electron beam emitter.

Although the present invention has been described with respect to an embodiment, it is to be understood that various modifications and changes may be made without departing from the object and scope of the invention as defined in the appended claims.

In the Figure 5a embodiment two second electron beam emitters are shown opposite each other. In an alternative embodiment there is only one second electron beam emitter, and the packaging container is made to rotate approximately one round around its own longitudinal axis when passing through the interface region. In order to ensure an aseptic lock, the opening between the sterilization chamber and the aseptic chamber should not be larger than that the electron cloud, generated by the single second electron beam emitter, can cover the opening.

The sterilization device has been described and illustrated in a schematic way in the above described embodiments and in the drawings. Only parts of the sterilization device being involved in the invention has been described, but it is to be understood that the sterilization device comprises also additional parts such as drive units for driving the first and second conveying means, and irradiation shielding enclosing the sterilization device for securing that electrons and x-rays are not spread to the environment outside of the device.

## Claims

1. Sterilization device for sterilizing packaging containers (12) with electron beams in a filling machine, said sterilization device comprising
at least one first electron beam emitter (10) adapted for sterilization of at least the interior of the packaging container (12),
at least one second electron beam emitter (36) adapted for sterilization of at least the exterior of said packaging container (12), a sterilization chamber (60) in which the first electron beam emitter (10) is adapted to sterilize the interior of the packaging container, and wherein
the at least one second electron beam emitter (36) is positioned such that an electron exit window thereof is facing an interface region (64), said interface region (64) forming an opening from the sterilization chamber (60) to an aseptic chamber (62), and wherein an electron cloud (I; II) emitted from the at least one second electron beam emitter (36) during operation is adapted to form an aseptic lock at least covering the interface region (64),
the sterilization chamber (60) comprises at least first conveying means, and the aseptic chamber (62) comprises at least second conveying means, and that the first and second conveying means are separately arranged one on each side of the interface region (64),
the first conveying means is adapted to push the packaging container (12) at least partly through the aseptic lock in the interface region (64), and release the packaging container when it has been received by the second conveying means on the other side of the aseptic lock, whereby the packaging container (12), when entering the aseptic chamber (62) has an open bottom end (34) and is held upside down.

2. Sterilization device according to claim 1, wherein an electron cloud (III) emitted from the first electron beam emitter (10), upon finalisation of the interior sterilization of the packaging container (12), is adapted to be partly overlapping the electron cloud (I; II) emitted from the at least one second electron beam emitter (36).

3. Sterilization device according to any of the preceding claims, wherein the first electron beam emitter (10) and the packaging container (12) are adapted to perform a mutual relative movement during which the interior sterilization of the packaging container (12) takes place.

4. Sterilization device according to claim 3, wherein the first conveying means is adapted to displace the packaging container (12) in relation to the first electron beam emitter (10) between a first position in which the packaging container (12) and the first electron beam emitter (10) are not engaged with each other and a second position in which the packaging container (12) and the first electron beam emitter (10) are fully engaged with each other.

5. Sterilization device according to any of the preceding claims, wherein said at least one second electron beam emitter (36) being adapted to sterilize the exterior of the packaging container (12) when passing the packaging container from the sterilization chamber (60) to the aseptic chamber (62), through the interface region (64), such that the packaging container reaching the aseptic chamber (62) is fully sterilized on its exterior.

6. Sterilization device according to any of the preceding claims, wherein it comprises two second electron beam emitters (36), arranged opposite each other with their electron exit windows (40) facing each other and the interface region (64), in such a way that the packaging containers (12) can pass in between them.

7. Sterilization device according to any of the claims 1-6, wherein the first electron beam emitter (10) is arranged in the first conveying means, and wherein the first conveying means is adapted to convey the packaging container (12) synchronously with the first electron beam emitter (10), keeping a longitudinal axis of the first electron beam emitter (10) aligned with a longitudinal axis of the packaging container (12).

8. Sterilization device according to claim 7, wherein the first conveying means is adapted to displace the packaging container (12) from a sterilization chamber in-feed position (68; 78) to an out-feed position, the out-feed position being the interface region (64), and that the first electron beam emitter (10) is adapted to sterilize the interior of the packaging container (12) at least during a portion of the displacement between the sterilization in-feed position (68; 78) to the out-feed position.

9. Sterilization device according to any of claims 1-8, wherein the first conveying means is provided with more than one first electron beam emitter (10).

10. Sterilization device according to any of the preceding claims, wherein the aseptic chamber (62) comprises stations in which the packaging container is adapted to be filled with product and sealed.

11. Method of sterilizing packaging containers (12) with electron beams in a filling machine, said method comprising
sterilizing at least the interior of the packaging container (12) with a first electron beam emitter (10) arranged in a sterilization chamber (60),
sterilizing at least the exterior of the packaging container (12) with at least one second electron beam emitter (36), and
wherein
the step of sterilizing the exterior of the packaging container is performed in an interface region (64), said interface region (64) forming an opening from the sterilization chamber (60) to an aseptic chamber (62), and wherein an electron cloud (I; II) emitted from the at least one second electron beam emitter (36) during operation is adapted to form an aseptic lock at least covering the interface region (64), and the sterilization chamber (60) comprises at least first conveying means, and the aseptic chamber (62) comprises at least second conveying means, and that the first and second conveying means are separately arranged one on each side of the interface region (64), and said method further comprises
the step of pushing the packaging container at least partly through the aseptic lock in the interface region (64), with the first conveying means and releasing the packaging container when it has been received by the second conveying means on the other side of the aseptic lock,
whereby the packaging container (12), when entering the aseptic chamber (62) has an open bottom end (34) and is held upside down.

12. Method according to claim 11, wherein the method comprises the step of finalizing the interior sterilization such that an electron cloud (III) emitted from the first electron beam emitter (10) partly overlaps the electron cloud (I; II) emitted from the at least one second electron beam emitter (36).

## Patentansprüche

1. Sterilisationsvorrichtung zum Sterilisieren von Verpackungsbehältern (12) mit Elektronenstrahlen in einer Abfüllmaschine, wobei die Sterilisationsvorrichtung umfasst:
wenigstens einen ersten Elektronenstrahlemitter (10), ausgelegt zum Sterilisieren wenigstens des Inneren des Verpackungsbehälters (12),
wenigstens einen zweiten Elektronenstrahlemitter (36), ausgelegt zum Sterilisieren wenigstens des Äußeren des Verpackungsbehälters (12), eine Sterilisationskammer (60), in der der erste Elektronenstrahlemitter (10) dafür ausgelegt ist, das Innere des Verpackungsbehälters zu sterilisieren, und wobei
der wenigstens eine zweite Elektronenstrahlemitter (36) so angeordnet ist, dass ein Elektronenaustrittfenster davon einem Übergangsbereich (64) zugewandt ist, wobei der Übergangsbereich (64) eine Öffnung von der Sterilisationskammer (60) zu einer aseptischen Kammer (62) bildet und wobei eine während des Betriebs von dem wenigstens einen zweiten Elektronenstrahlemitter (36) emittierte Elektronenwolke (I; II) dafür ausgelegt ist, eine aseptische Sperre zu bilden, die wenigstens den Übergangsbereich (64) abdeckt,
die Sterilisationskammer (60) wenigstens ein erstes Beförderungsmittel umfasst und die aseptische Kammer (62) wenigstens ein zweites Beförderungsmittel umfasst und das erste und das zweite Beförderungsmittel getrennt an jeder Seite des Übergangsbereichs (64) angeordnet sind,
das erste Beförderungsmittel dafür ausgelegt ist, den Verpackungsbehälter (12) wenigstens teilweise durch die aseptische Sperre in dem Übergangsbereich (64) zu drücken und den Verpackungsbehälter freizugeben, wenn er von dem zweiten Beförderungsmittel an der anderen Seite der aseptischen Sperre empfangen worden ist, wobei der Verpackungsbehälter (12) beim Eintreten in die aseptische Kammer (62) ein offenes Bodenende (34) aufweist und auf dem Kopf stehend gehalten wird.

2. Sterilisationsvorrichtung gemäß Anspruch 1, wobei eine von dem ersten Elektronenstrahlemitter (10) emittierte Elektronenwolke (III) dafür ausgelegt ist, nach dem Abschluss der Innensterilisation des Verpackungsbehälters (12) teilweise mit der von dem wenigstens einen zweiten Elektronenstrahlemitter (36) emittierten Elektronenwolke (I, II) zu überlappen.

3. Sterilisationsvorrichtung gemäß einem der vorstehenden Ansprüche, wobei der erste Elektronenstrahlemitter (10) und der Verpackungsbehälter (12) dafür ausgelegt sind, eine gegenseitige Relativbewegung durchzuführen, während derer die Innensterilisation des Verpackungsbehälters (12) erfolgt.

4. Sterilisationsvorrichtung gemäß Anspruch 3, wobei das erste Beförderungsmittel dafür ausgelegt ist, den Verpackungsbehälter (12) relativ zu dem ersten Elektronenstrahlemitter (10) zwischen einer ersten Position, in der der Verpackungsbehälter (12) und der erste Elektronenstrahlemitter (10) nicht miteinander in Eingriff stehen, und einer zweiten Position, in der der Verpackungsbehälter (12) und der erste Elektronenstrahlemitter (10) vollständig miteinander in Eingriff stehen, zu bewegen.

5. Sterilisationsvorrichtung gemäß einem der vorstehenden Ansprüche, wobei der wenigstens eine zweite Elektronenstrahlemitter (36) dafür ausgelegt ist, das Äußere des Verpackungsbehälters (12) zu sterilisieren, wenn sich der Verpackungsbehälter von der Sterilisationskammer (60) zu der aseptischen Kammer (62) durch den Übergangsbereich (64) bewegt, so dass der Verpackungsbehälter, wenn er die aseptische Kammer (62) erreicht, an seinem Äußeren vollständig sterilisiert ist.

6. Sterilisationsvorrichtung gemäß einem der vorstehenden Ansprüche, wobei sie zwei zweite Elektronenstrahlemitter (36) umfasst, die einander gegenüberstehend angeordnet sind, wobei ihre Elektronenaustrittsfenster (40) einander und dem Übergangsbereich (64) auf eine solche Weise zugewandt sind, dass die Verpackungsbehälter (12) zwischen ihnen durchtreten können.

7. Sterilisationsvorrichtung gemäß einem der Ansprüche 1-6, wobei der erste Elektronenstrahlemitter (10) in dem ersten Beförderungsmittel angeordnet ist und wobei das erste Beförderungsmittel dafür ausgelegt ist, den Verpackungsbehälter (12) synchron mit dem ersten Elektronenstrahlemitter (10) zu bewegen, wobei die Längsachse des ersten Elektronenstrahlemitters (10) mit der Längsachse des Verpackungsbehälters (12) ausgerichtet bleibt.

8. Sterilisationsvorrichtung gemäß Anspruch 7, wobei das erste Beförderungsmittel dafür ausgelegt ist, den Verpackungsbehälter (12) aus einer Sterilisationskammer-Einführposition (68; 78) zu einer Ausführposition zu bewegen, wobei die Ausführposition der Übergangsbereich (64) ist, und wobei der erste Elektronenstrahlemitter (10) dafür ausgelegt ist, das Innere des Verpackungsbehälters (12) wenigstens während eines Teils der Bewegung zwischen der Sterilisations-Einführposition (68; 78) zu der Ausführposition zu sterilisieren.

9. Sterilisationsvorrichtung gemäß einem der Ansprüche 1-8, wobei das erste Beförderungsmittel mit mehr als einem ersten Elektronenstrahlemitter (10) ausgestattet ist.

10. Sterilisationsvorrichtung gemäß einem der vorstehenden Ansprüche, wobei die aseptische Kammer (62) Stationen umfasst, wobei der Verpackungsbehälter dafür ausgelegt ist, darin mit Produkt gefüllt und verschlossen zu werden.

11. Verfahren zum Sterilisieren von Verpackungsbehältern (12) mit Elektronenstrahlen in einer Abfüllmaschine, wobei das Verfahren umfasst:
Sterilisieren wenigstens des Inneren des Verpackungsbehälters (12) mit einem ersten Elektronenstrahlemitter (10), der in einer Sterilisationskammer (60) angeordnet ist, Sterilisieren wenigstens des Äußeren des Verpackungsbehälters (12) mit wenigstens einem zweiten Elektronenstrahlemitter (36) und
wobei
der Schritt des Sterilisierens des Äußeren des Verpackungsbehälters in einem Übergangsbereich (64) durchgeführt wird, wobei der Übergangsbereich (64) eine Öffnung von der Sterilisationskammer (60) zu einer aseptischen Kammer (62) bildet und wobei eine während des Betriebs von dem wenigstens einen zweiten Elektronenstrahlemitter (36) emittierte Elektronenwolke (I; II) dafür ausgelegt ist, eine aseptische Sperre zu bilden, die wenigstens den Übergangsbereich (64) abdeckt, und die Sterilisationskammer (60) wenigstens ein erstes Beförderungsmittel umfasst und die aseptische Kammer (62) wenigstens ein zweites Beförderungsmittel umfasst und das erste und das zweite Beförderungsmittel getrennt an jeder Seite des Übergangsbereichs (64) angeordnet sind und das Verfahren ferner umfasst:
den Schritt des Drückens des Verpackungsbehälters wenigstens teilweise durch die aseptische Sperre in dem Übergangsbereich (64) durch das erste Beförderungsmittel und Freigeben des Verpackungsbehälters, wenn er von dem zweiten Beförderungsmittel an der anderen Seite der aseptischen Sperre empfangen worden ist,
wobei der Verpackungsbehälter (12) beim Eintreten in die aseptische Kammer (62) ein offenes Bodenende (34) aufweist und auf dem Kopf stehend gehalten wird.

12. Verfahren gemäß Anspruch 11, wobei das Verfahren den Schritt des derartigen Abschließens der Innensterilisation umfasst, dass eine von dem ersten Elektronenstrahlemitter (10) emittierte Elektronenwolke (III) teilweise mit der von dem wenigstens einen zweiten Elektronenstrahlemitter (36) emittierten Elektronenwolke (I, II) überlappt.

## Revendications

1. Dispositif de stérilisation destiné à stériliser des récipients d'emballage (12) avec des faisceaux d'électrons dans une machine à remplir, ledit dispositif de stérilisation comprenant
au moins un premier émetteur de faisceau d'électrons (10) adapté pour stériliser au moins l'intérieur du récipient d'emballage (12),
au moins un deuxième émetteur de faisceau d'électrons (36) adapté pour stériliser au moins l'extérieur dudit récipient d'emballage (12),
une chambre de stérilisation (60) dans laquelle le premier émetteur de faisceau d'électrons (10) est adapté pour stériliser l'intérieur du récipient d'emballage, et dans lequel
l'au moins un deuxième émetteur de faisceau d'électrons (36) est positionné de telle sorte qu'une fenêtre de sortie d'électrons de celui-ci fait face à une région d'interface (64), ladite région d'interface (64) formant une ouverture depuis la chambre de stérilisation (60) jusqu'à une chambre aseptique (62), et un nuage d'électrons (I ; II) émis depuis l'au moins un deuxième émetteur de faisceau d'électrons (36) en fonctionnement étant adapté pour former un sas aseptique recouvrant au moins la région d'interface (64),
la chambre de stérilisation (60) comprend au moins un premier moyen de transport, et la chambre aseptique (62) comprend au moins un deuxième moyen de transport, et les premier et deuxième moyens de transport sont disposés séparément sur chaque côté de la région d'interface (64),
le premier moyen de transport est adapté pour pousser le récipient d'emballage (12) au moins partiellement à travers le sas aseptique dans la région d'interface (64), et libérer le récipient d'emballage quand il a été reçu par le deuxième moyen de transport sur l'autre côté du sas aseptique, le récipient d'emballage (12), lorsqu'il pénètre dans la chambre aseptique (62), ayant une extrémité inférieure ouverte (34) et étant maintenu à l'envers.

2. Dispositif de stérilisation selon la revendication 1, dans lequel un nuage d'électrons (III) émis depuis le premier émetteur de faisceau d'électrons (10), lors de la finalisation de la stérilisation intérieure du récipient d'emballage (12), est adapté pour chevaucher partiellement le nuage d'électrons (I ; II) émis depuis l'au moins un deuxième émetteur de faisceau d'électrons (36).

3. Dispositif de stérilisation selon l'une quelconque des revendications précédentes, dans lequel le premier émetteur de faisceau d'électrons (10) et le récipient d'emballage (12) sont adaptés pour effectuer un mouvement relatif mutuel pendant lequel a lieu la stérilisation intérieure du récipient d'emballage (12).

4. Dispositif de stérilisation selon la revendication 3, dans lequel le premier moyen de transport est adapté pour déplacer le récipient d'emballage (12) par rapport au premier émetteur de faisceau d'électrons (10) entre une première position dans laquelle le récipient d'emballage (12) et le premier émetteur de faisceau d'électrons (10) ne sont pas engagés l'un avec l'autre et une deuxième position dans laquelle le récipient d'emballage (12) et le premier émetteur de faisceau d'électrons (10) sont pleinement engagés l'un avec l'autre.

5. Dispositif de stérilisation selon l'une quelconque des revendications précédentes, dans lequel ledit au moins un deuxième émetteur de faisceau d'électrons (36) est adapté pour stériliser l'extérieur du récipient d'emballage (12) lors du passage du récipient d'emballage de la chambre de stérilisation (60) à la chambre aseptique (62), par la région d'interface (64), de telle sorte que le récipient d'emballage atteignant la chambre aseptique (62) est entièrement stérilisé sur son extérieur.

6. Dispositif de stérilisation selon l'une quelconque des revendications précédentes, qui comprend deux deuxièmes émetteurs de faisceau d'électrons (36), disposés à l'opposé l'un de l'autre avec leurs fenêtres de sortie d'électrons (40) se faisant face et faisant face à la région d'interface (64), de telle sorte que les récipients d'emballage (12) peuvent passer entre ceux-ci.

7. Dispositif de stérilisation selon l'une quelconque des revendications 1 à 6, dans lequel le premier émetteur de faisceau d'électrons (10) est disposé dans le premier moyen de transport, et dans lequel le premier moyen de transport est adapté pour transporter le récipient d'emballage (12) de façon synchrone avec le premier émetteur de faisceau d'électrons (10), en maintenant un axe longitudinal du premier émetteur de faisceau d'électrons (10) aligné avec un axe longitudinal du récipient emballage (12).

8. Dispositif de stérilisation selon la revendication 7, dans lequel le premier moyen de transport est adapté pour déplacer le récipient d'emballage (12) d'une position d'entrée de la chambre de stérilisation (68 ; 78) à une position de sortie, la position de sortie étant la région d'interface (64), et le premier émetteur de faisceau d'électrons (10) est adapté pour stériliser l'intérieur du récipient d'emballage (12) au moins pendant une partie du déplacement entre la position d'entrée de la stérilisation (68 ; 78) et la position de sortie.

9. Dispositif de stérilisation selon l'une quelconque des revendications 1 à 8, dans lequel le premier moyen de transport est pourvu de plusieurs premiers émetteurs de faisceau d'électrons (10).

10. Dispositif de stérilisation selon l'une quelconque des revendications précédentes, dans lequel la chambre aseptique (62) comprend des stations dans lesquelles le récipient d'emballage est adapté pour être rempli avec un produit et scellé.

11. Procédé de stérilisation de récipients d'emballage (12) avec des faisceaux d'électrons dans une machine à remplir, ledit procédé comprenant les étapes suivantes :
stériliser au moins l'intérieur du récipient d'emballage (12) avec un premier émetteur de faisceau d'électrons (10) disposé dans une chambre de stérilisation (60),
stériliser au moins l'extérieur du récipient d'emballage (12) avec au moins un deuxième émetteur de faisceau d'électrons (36), et
dans lequel
l'étape de stérilisation de l'extérieur du récipient d'emballage est effectuée dans une région d'interface (64), ladite région d'interface (64) formant une ouverture depuis la chambre de stérilisation (60) jusqu'à une chambre aseptique (62), et un nuage d'électrons (I ; II) émis depuis l'au moins un deuxième émetteur de faisceau d'électrons (36) en fonctionnement étant adapté pour former un sas aseptique recouvrant au moins la région d'interface (64), et la chambre de stérilisation (60) comprend au moins un premier moyen de transport, et la chambre aseptique (62) comprend au moins un deuxième moyen de transport, et les premier et deuxième moyens de transport sont disposés séparément sur chaque côté de la région d'interface (64), et ledit procédé comprend en outre
l'étape consistant à pousser le récipient d'emballage au moins partiellement à travers le sas aseptique dans la région d'interface (64) avec le premier moyen de transport, et libérer le récipient d'emballage quand il a été reçu par le deuxième moyen de transport sur l'autre côté du sas aseptique,
le récipient d'emballage (12), lorsqu'il pénètre dans la chambre aseptique (62), ayant une extrémité inférieure ouverte (34) et étant maintenu à l'envers.

12. Procédé selon la revendication 11, le procédé comprenant l'étape consistant à finaliser la stérilisation intérieure de telle sorte qu'un nuage d'électrons (III) émis depuis le premier émetteur de faisceau d'électrons (10) chevauche partiellement le nuage d'électrons (I ; II) émis depuis l'au moins un deuxième émetteur de faisceau d'électrons (36).
